# EUROPÄISCHE PATENTSCHRIFT

(11) **EP 1 976 825 B1**
(45) Veröffentlichungstag und Bekanntmachung des Hinweises auf die Patenterteilung: **08.06.2011**
(21) Anmeldenummer: 07703734.9
(22) Anmeldetag: 09.01.2007
(51) Int. Cl.: C07C 263/06, C07C 265/14

(54) **VERFAHREN ZUR HERSTELLUNG VON ISOCYANATEN**
METHOD FOR PRODUCING ISOCYANATES
PROCEDE DE PRODUCTION D'ISOCYANATES

(30) Priorität: 13.01.2006 EP 06100315
(43) Veröffentlichungstag der Anmeldung: 08.10.2008
(73) Patentinhaber: BASF SE, 67056 Ludwigshafen (DE)
(72) Erfinder: KLÖTZER, Matthias, 01945 Kroppen (DE); STROEFER, Eckhard, 68163 Mannheim (DE); BLANKERTZ, Heinrich-Josef, 67147 Forst (DE)
(86) Internationale Anmeldenummer: PCT/EP2007/050184
(87) Internationale Veröffentlichungsnummer: WO 2007/082818

(56) Entgegenhaltungen:
- EP-A- 1 512 682
- EP-A1- 0 018 588
- WO-A-98/54129
- DE-C- 748 714
- US-A- 2 145 242

## Beschreibung

Gegenstand der Erfindung ist ein mehrstufiges Verfahren zur kontinuierlichen Herstellung von organischen, destillierbaren Polyisocyanaten, vorzugsweise von Diisocyanaten, besonders bevorzugt von aliphatischen oder cycloaliphatischen Diisocyanaten, durch Umsetzung der entsprechenden organischen Polyamine mit Harnstoffen in niedermolekulare monomere Polyharnstoffe und deren thermische Spaltung.

Die technischen Verfahren zur Herstellung von organischen Polyisocyanaten, wie z.B. von aromatischen, aliphatischen oder cycloaliphatischen Polyisocyanaten, beruhen auf der Phosgenierung der entsprechenden organischen Polyamine zu Polycarbamidsäurechloriden und deren thermische Spaltung zu den Polyisocyanaten und Chlorwasserstoff. Abgesehen von den schwerwiegenden Umweltschutz-, Entsorgungs- und Sicherheitsproblemen, die der Einsatz von Phosgen mit sich bringt, sind diese Verfahren mit weiteren entscheidenden Nachteilen behaftet. So gelingt die Herstellung von aliphatischen oder cycloaliphatischen Polyisocyanaten aufgrund der stärkeren Basizität der Ausgangspolyamine nur mit recht mäßigen Raum-Zeit-Ausbeuten. Nachteilig ist ferner die Bildung von unerwünschten Nebenprodukten, die, bereits in Spuren vorliegend, zu starken Verfärbungen der Polyisocyanate führen können. Bei der Hexamethylendiisocyanat-1,6 (HDI)-Herstellung entstehen z.B. mehrere Nebenprodukte, von denen das wichtigste, 6-Chlorhexylisocyanat, zudem den Nachteil besitzt, daß es nur mit erheblichem destillativen Aufwand vom HDI abgetrennt werden kann.

Problematisch bei dieser Verfahrensweise sind insbesondere der hohe Umsatz von Chlor über Phosgen und Carbamidsäurechlorid in Chlorwasserstoff, die Toxizität des Phosgens sowie die Korrosivität des Reaktionsgemisches, die Labilität der in der Regel eingesetzten Lösungsmittel und die Bildung halogenhaltiger Rückstände.

Obwohl die thermische Spaltung von (cyclo)aliphatischen und insbesondere aromatischen Mono- und Diurethanen in die entsprechenden Isocyanate und Alkohol seit langem bekannt ist, sind es besonders die unerwünschten Nebenreaktionen und vor allem die Tendenz der Reaktionsmischungen zur Ausbildung von Belegungen, Verharzungen und Verstopfungen in Reaktoren und Aufarbeitungseinrichtungen, die die Wirtschaftlichkeit der Prozesse nachhaltig beeinträchtigen.

In den vergangenen Jahrzehnten hat es deshalb viele Anstrengungen gegeben, diese Nachteile des Verfahrens durch ein einfacheres und verbessertes Verfahren zu beseitigen. So wurden zur Herstellung von aliphatischen und/oder cycloaliphatischen Di- und/oder Polyurethanen gemäß EP 18588 A1 oder auch wie in EP 28338 A2 primäre aliphatische und/oder cycloaliphatische Di- und/oder Polyamine mit O-Alkylcarbamidsäureestern in Gegenwart von Alkoholen bei Temperaturen von 160 bis 300 °C mit und ohne Katalysator umgesetzt. Die entstandenen Di- und/oder Polyurethane können in die entsprechenden Isocyanate überführt werden. Der bei der Umsetzung der Amine entstehende Ammoniak kann dabei abgetrennt werden.

Weitere Veröffentlichungen befassen sich mit der teilweisen Substitution von Harnstoff und/oder Diaminen durch carbonylgruppenhaltige Verbindungen (z.B. EP 27952 oder EP 126299). Das phosgenfreie Verfahren wird ausführlich beispielsweise in EP 566925 A2 beschrieben.

Nachteilig am letzteren Verfahren ist, daß die intermediär gebildeten Polyharnstoffe in einem weiteren Reaktionsschritt in die korrespondierenden Carbamate überführt werden müssen, die dann gespaltet werden können. Eine direkte Spaltung der direkt erhaltenen Polyharnstoffe ist nicht möglich, da die Spaltungsprodukte, Amin und Isocyanat, zumeist noch in der Gasphase des Spaltungsreaktors aber spätestens bei der Bildung von Kondensat miteinander reagieren.

Die thermische Zersetzung von Harnstoffen zur Herstellung von Isocyanaten ist prinzipiell bekannt, s. z.B. Houben-Weyl, Methoden der organischen Chemie, Band VIII, S. 126 bis 128, 1952. Die dort beschriebene Methode ist jedoch nur für das hochsiedende Diphenylamin präparativ anwendbar. Zudem muß Diphenylamin in einem vorgeschalteten Schritt mit Phosgen in das korrespondierende Diphenylcarbamidsäurechlorid überführt werden, so daß diese Reaktion mithin nicht phosgenfrei verläuft.

Zur Thermolyse geeignet sind nach dem Verfahren aus der DT-PS 748 714 jedoch lediglich trisubstituierte, höchstens zwei Arylgruppen aufweisende Harnstoffe. Nachteilig bei diesem Verfahren ist, dass die beiden Amidgruppen dieser Harnstoffe zwei Aminen von erheblich verschiedenen Siedepunkten entsprechen müssen, wobei nur niedrigsiedende Isocyanate der Rekombination durch Abdestillieren entzogen werden können.

WO 98/54129 offenbart die Spaltung von trisubstituierten Harnstoffen, so daß sekundäre Amine freigesetzt werden. Zudem müssen die Harnstoffe noch mindestens ein tertiäres Kohlenstoffatom aufweisen. Dieses Substitutionsmuster ist notwendig, um aus dem trisubstituierten Harnstoff selektiv nur das sekundäre Amin abzuspalten und die NCO-Gruppe aus der primär substituierten Harnstoffgruppe herauszubilden. Bei der Spaltung eines unsymmetrisch N,N'-disubstituierten Harnstoffs bestünde die Gefahr, daß sich unselektiv verschiedene Isocyanate bilden.

Mithin sind die aus dem Stand der Technik bekannten Verfahren zur Spaltung von Harnstoffen nur mit speziellen substituierten Harnstoffen durchführbar.

Die aus dem Stand der Technik vorbekannten Verfahren sind also lediglich zur Herstellung von Monoisocyanaten geeignet, nicht jedoch zur Umsetzung von Di- oder Polyaminen.

Die Aufgabe der vorliegenden Erfindung bestand darin, ein Verfahren bereitzustellen, mit dem organische Polyisocyanate durch Spaltung der korrespondierenden Polyharnstoffe hergestellt werden können.

Diese Aufgabe konnte gelöst werden durch ein Verfahren zur Herstellung von Isocyanaten durch Umsetzung mindestens eines Di- oder Polyamins mit Harnstoff zum korrespondierenden Di- oder Polyharnstoff und anschließender thermischer Spaltung der so erhaltenen Di- oder Polyharnstoffe in die korrespondierenden Isocyanate.

Gegenstand der Erfindung ist ein mehrstufiges Verfahren zur kontinuierlichen Herstellung von organischen Di- oder Polyisocyanaten durch Umsetzung der entsprechenden primären organischen Di- oder Polyamine mit Harnstoff in die entsprechenden Di- oder Polyharnstoffe und deren thermische Spaltung, in dem man
a) mindestens ein organisches Di- oder Polyamin mit Harnstoff in Gegenwart oder vorzugsweise in Abwesenheit mindestens eines Katalysators und in Abwesenheit eines Alkohols zu den korrespondierenden Di- oder Polyharnstoffen umsetzt,
b) den dabei entstehenden Ammoniak abtrennt,
c) aus dem Austrag aus a) oder b) überschüssigen Harnstoff und gegebenenfalls weitere Nebenkomponenten abtrennt,
d) das vom Harnstoff und gegebenenfalls weiteren Nebenkomponenten befreite Di- oder Polyharnstoffe enthaltende Reaktionsgemisch aus c) zumindest teilweise einer thermischen Spaltung zuführt, in der man das Reaktionsgemisch in einer Spalteinrichtung in das entsprechende Di- oder Polyisocyanat und Ammoniak spaltet,
e) das aus (d) erhaltene Rohisocyanat in mindestens einer Destillation reinigt und anfallende Destillationsrückstände zumindest teilweise erneut der Spaltung (d) zuführt und/oder mit Ammoniak und/oder Wasser zu Di- oder Polyharnstoff und/oder Amin umwandelt und der Reaktionseinheit (a) und/oder (b) zuführt.

Das erfindungsgemäße Verfahren weist höhere Ausbeuten als im Stand der Technik bekannte Verfahren auf.

Rein formal betrachtet kann das erfindungsgemäße Verfahren schematisch durch folgende Gleichung bilanziert werden:

R-(NH₂)ₙ + n H₂N(CO)NH₂ → R(NCO)ₙ + 2n NH₃

Als Di- und Polyamine R-(NH₂)ₙ, hier kurz als Amin bezeichnet, eignen sich Amine der Formel R(NH₂)ₙ, in der R einen n-wertigen, vorzugsweise zweiwertigen organischen Rest, wie z.B. einen gegebenenfalls substituierten, beispielsweise mit einer Alkylgruppe substituierten aromatischen oder vorzugsweise einen linearen oder verzweigtkettigen, aliphatischen oder gegebenenfalls substituierten cycloaliphatischen Rest bedeutet.

Der Index n bedeutet darin eine positive ganze Zahl von 2 oder mehr, bevorzugt 2, 3 oder 4, besonders bevorzugt 2 oder 3 und ganz besonders bevorzugt 2.

Als geeignete aromatische Polyamine beispielhaft genannt seien 2,4- und 2,6-Toluylen-diamin, 4,4'-, 2,4'- und 2,2'-Diamino-diphenylmethane und die entsprechenden Isomerengemische.

Als aliphatische oder cycloaliphatische Polyamine kommen beispielsweise in Betracht: Butandiamin-1,4, 2-Ethylbutandiamin-1,4, Octandiamin-1,8, Decandiamin-1,10, Dodecandiamin-1,12, Cyclohexandiamin-1,4, 2-Methyl-, 4-Methyl-cyclohexandiamin-1,3, 1,3- und 1,4-Diaminomethylcyclohexan, 4,4'-Di(aminocyclohexyl)methan, 3 (bzw. 4), 8 (bzw. 9)-Bis(aminomethyl)-tricyclo[5.2.1.0²⁶]decan-Isomerengemische. Vorzugsweise Verwendung finden 2-Methylpentandiamin-1,5, 2,2,4- bzw. 2,4,4-Trimethylhexandiamin-1,6 und insbesondere Hexandiamin-1,6 und 3-Aminomethyl-3,5,5-trimethylcyclohexylamin.

Mit Harnstoff ist in dieser Schrift unsubstituierter Harnstoff H₂N-(CO)-NH₂ bezeichnet. Der Biuretanteil im Harnstoff spielt erfindungsgemäß eine untergeordnete Rolle und kann bevorzugt bis zu 1 Gew%, besonders bevorzugt bis zu 0,5 Gew% und ganz besonders bevorzugt bis zu 0,3 Gew% betragen.

Die einzelnen Stufen des Verfahrens werden im Folgenden beschrieben:

### a) Umsetzung des Amins mit Harnstoff

Zur Herstellung der Di- oder Polyharnstoffe in der Reaktionsstufe (a) werden die korrespondierenden Amine mit Harnstoff zweckmäßigerweise in einem molaren Verhältnis von Amin zu Harnstoff von 1:50 bis 1:2, bevorzugt 1:30 bis 1:3, besonders bevorzugt 1 : 20 bis 1 : 5 und ganz besonders bevorzugt 1:15 - 1:8 bei Temperaturen von 50 - 300 °C und insbesondere bei 180 - 220 °C unter einem Druck von 0,1 bis 30 bar, vorzugsweise 1-20 bar zur Reaktion gebracht. Für diese Reaktionsbedingungen ergeben sich mittlere Reaktionszeiten von Bruchteilen von Sekunden bis Minuten, bevorzugt 0,1 s-5 min, bevorzugt 0,5 s - 3 min.

Die Umsetzung in der Reaktionsstufe (a) kann auch in Gegenwart von Katalysatoren erfolgen. Diese werden zweckmäßiger Weise in Mengen von 0,001 bis 20 Gew% vorzugsweise 0,002 bis 5 Gew% insbesondere 0,005 bis 0,1 Gew%, bezogen auf das Gewicht des Amins, eingesetzt.

Als Katalysatoren eignen sich anorganische oder organische Verbindungen; die ein oder mehrere Kationen, vorzugsweise ein Kation von Metallen der Gruppe IA; IB, IIA, IIB, IIIB, IVA, IVB, VA, VB, VIB, VIIB, VIIIB des Periodensystems der Elemente enthalten, definiert gemäß Handbook of Chemistry and Physics 14th Edition, publiziert von Chemical Rubber Publishing Co., 23 Superior Ave. N.E., Cleveland, Ohio, enthalten. Beispielhaft genannt seien die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen und Cobalt.

Der Katalysator kann weiterhin mindestens ein Anion enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocarbamate.

Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt: Lithiummethanolat, Lithiumethanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminiumacetylacetonat, Aluminium-iso-butylat, Aluminiumtrichlorid, Bismut-(III)-chlorid, Kupfer-(II)-acetat, Kupfer-(II)-sulfat, Kupfer-(II)-nitrat, Bis-(triphenylphosphinoxido)-kupfer-(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylenat, Cer-(IV)-oxid, Uranylacetat, Titantetrabutanolat, Titantetrachlorid, Titantetraphenolat, Titannaphtenat, Vanadium-(III)-chlorid, Vanadiumacetylacetonat, Chrom-(III)-chlorid, Molybdän-(VI)-oxid, Molybdänacetylacetonat, Wolfram-(VI)-oxid, Mangan-(II)-chlorid, Mangan-(II)-acetat, Mangan-(III)-acetat, Eisen-(II)-acetat, Eisen-(III)-acetat, Eisenphosphat, Eisenoxalat, Eisen-(III)-chlorid, Eisen-(III)-bromid, Cobaltacetat, Cobaltchlorid, Cobaltsulfat, Cobaltnaphthenat, Nickelchlorid, Nickelacetat und Nickelnaphthenat sowie deren Gemische.

Als bevorzugte Katalysatoren seien beispielhaft folgende Verbindungen genannt: Lithiumbutanolat, Aluminiumacetylacetonat, Zinkacetylacetonat, Titantetrabutanolat und Zirkontetrabutylat.

Für die Reaktion werden Amin und Harnstoff sowie gegebenenfalls Lösungsmittel sowie gegebenenfalls Katalysator bevorzugt in eine Mischeinrichtung geführt und innig miteinander vermischt.

Als Mischeinrichtung bevorzugt wird ein Mischkreis, ein Rührbehälter, eine Mischpumpe oder eine Düsenmischeinrichtung, beispielsweise Koaxialmischdüsen, Y- oder T-Mischer, oder eine Vortex-Impinging-Jet-Mischkonfiguration eingesetzt, bevorzugt ein Mischkreis, ein Rührbehälter, eine Mischpumpe oder eine Düsenmischeinrichtung.

Der Harnstoff wird bevorzugt flüssig in die Reaktion bzw. die Mischeinrichtung eindosiert. Weniger bevorzugt kann der Harnstoff aber auch als Paste des festen Harnstoffs in einem flüssigen Amin in die Reaktion geführt werden oder als Lösung in einem geeigneten Lösungsmittel, bevorzugt Wasser.

Als Reaktoren für die Reaktion können beispielsweise Rührkessel, Rührkesselkaskaden oder Rohrreaktoren dienen, sowie Reaktionsmischpumpen.

Die Reaktion wird bevorzugt unter Bedingungen ausgeführt, bei denen sich neben der flüssigen Reaktionsphase noch eine weitere Gasphase aus dem während der Reaktion gebildeten Ammoniak ausbilden kann. Weniger bevorzugt ist es, die Reaktion beispielsweise durch Anlegen von Druck einphasig zu halten.

Rohrreaktoren sollten bevorzugt weitestgehend rückvermischungsfrei sein. Dies wird beispielsweise erreicht durch das Verhältnis des Durchmessers des Rohrreaktors zu dessen Länge oder durch Einbauten, wie Lochböden, Schlitzböden oder statische Mischer. Bevorzugt wird die Rückvermischungsfreiheit durch das Verhältnis von Länge zu Durchmesser des Rohrreaktors erreicht.

Als Rohrreaktor eignen sich beispielsweise solche Rohre, mit denen die bereits beschriebene mittlere Verweilzeit des Reaktionsmediums erreicht und bei denen gleichzeitig eine turbulente Rohrströmung (Reynolds-Zahlen größer 2300) erreicht wird.

Die Bodensteinzahl des Rohrreaktors sollte größer als 5 sein, bevorzugt größer als 6, besonders bevorzugt größer als 10, ganz besonders bevorzugt von 10 bis 600 und insbesondere von 10 bis 100.

Der Rohrreaktor kann eine beliebige Orientierung im Raum aufweisen. Bevorzugt wird er als senkrechter Rohrreaktor aufgebaut, der besonders bevorzugt von unten nach oben durchströmt wird.

Der Rohrreaktor kann isotherm oder bevorzugt temperiert ausgeführt werden. Eine Temperierung kann durch eine Mantelheizung oder durch innenliegende Rohre oder Platten erfolgen. Die Beheizung erfolgt bevorzugt durch den Mantel.

Selbstverständlich kann der Rohrreaktor auch aus mehreren seriell geschalteten Rohrstücken bestehen, solange die Rückvermischungsfreiheit gewährleistet bleibt. Falls erforderlich können optional im Verlauf des Rohrreaktors, beispielsweise zwischen derartige Rohrstücke Phasenscheider zur Trennung von flüssiger und gasförmiger Phase vorgesehen werden, in denen während der Reaktion entstandener Ammoniak abgetrennt werden kann, so daß das Gleichgewicht der Reaktion verschoben wird.

Zur Vergrößerung der Produktionskapazität können erfindungsgemäß auch mehrere Rohrreaktoren parallel geschaltet werden.

Gegebenenfalls kann in den Rohrreaktor, wie oben ausgeführt, an einer oder mehreren Stellen, beispielsweise am Anfang und in der Mitte des Rohrreaktors, noch Harnstoff oder bevorzugt Amin nachdosiert werden.

Um die Gasbelastung für die Folgestufe gering zu halten, kann der Austrag aus dem Rohrreaktor in einer bevorzugten Ausführungsform einem Phasenscheider zugeführt werden und die dem Phasenscheider entnommene Flüssigphase dann der Folgestufe zugeführt werden.

Ein solcher Phasenscheider ist ein Behälter, in dem die Phasentrennung zwischen Gas- und Flüssigphase durch die Beruhigung der zweiphasigen, aus dem Gleichstromreaktor austretenden Strömung erreicht wird.

Der Phasenscheider kann isotherm oder bevorzugt beheizt ausgeführt werden, um das Ausfallen schwer löslicher Nebenprodukte zu verhindern. Die Beheizung kann beispielsweise über den Mantel oder über einen Kreislauf mit einem externen Wärmetauscher erfolgen. Bei Verwendung eines externen Wärmetauschers reicht eine normale Isolierung des Wärmetauschers.

Das Überführen des Reaktionsaustrages aus dieser Stufe in die nachfolgende Stufe kann vorteilhaft über Druckhalteventile erfolgen, wobei der Druck in dieser Stufe in der Regel mindestens 0,1 bar oberhalb des in Stufe b) herrschenden Druckes betragen sollte. Ist dies nicht der Fall, kann das Überführen z.B. mit Hilfe einer Pumpe oder barometrisch erfolgen.

Die Verweilzeit in dieser Stufe ist so gewählt, daß der Umsatz, bezogen auf Aminogruppen im eingesetzten Amin zu Harnstoffgruppen, nach Verlassen des Rohrreaktors mindestens 95%, bevorzugt mindestens 98, besonders bevorzugt mindestens 99 und ganz besonders bevorzugt mindestens 99,5% beträgt.

Der Austrag der Reaktionsmischung kann bei vollständigem Umsatz der Amine zum Di- oder Polyharnstoff direkt der Ammoniakabtrennung (b) zugeführt werden, wenn nicht bereits während der Reaktion erfolgt ist oder er wird zur Erzielung eines vollständigen Umsatzes einem weiteren Reaktor oder Reaktorsystem zugeführt. Als Reaktoren können weitere Rohrreaktoren, Mischreaktorkaskaden oder Kolonnen mit der notwendigen mittleren Verweilzeit zum Einsatz kommen.

Ist der Umsatz, bezogen auf Aminogruppen im eingesetzten Amin zu Harnstoffgruppen, nach Verlassen des Rohrreaktors-noch nicht-vollständig und beträgt beispielsweise weniger als 95%, so kann der Austrag nochmals nachreagiert werden.

Dazu kann das Reaktionsgemisch zur Vervollständigung des Umsatzes in einem weiteren Rohrreaktor oder aber auch in einem rückvermischten Reaktor nachreagieren gelassen werden, bevorzugt bis der Umsatz 98% oder mehr beträgt.

Unter rückvermischtem Reaktorsystem wird hier verstanden, dass die Bodensteinzahl des Reaktorsystems kleiner 5, bevorzugt kleiner 4 ist.

### b) Ammoniakabtrennung

Es hat sich als vorteilhaft erwiesen, den entstehenden Ammoniak sofort aus der Reaktionsmischung zu entfernen. Die dafür verwendete Vorrichtung, beispielsweise eine Destillationskolonne wird bei Temperaturen von 50 -180 °C betrieben. Die Temperatur richtet sich einerseits im wesentlichen nach dem Schmelzpunkt des in der Reaktionstufe (a) entstandenen Reaktionsproduktes, andererseits nach dem Siedepunkt des verwendeten Lösungsmittels.

Zur Abtrennung des Ammoniaks werden zweckmäßigerweise Kolonnen verwendet, bevorzugt wird der Ammonik per Destillation abgetrennt. Üblicherweise erfolgt die Abtrennung in einem Druckbereich von 0,005 - 20 bar, vorzugsweise bei 0,04 - 15 bar. Die notwendigen Temperaturen betragen beispielsweise bei 50 -180 °C, bevorzugt bei 80 bis 160 °C.

Diese Destillationseinheit ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt. Bevorzugt werden Böden verwendet, besonders bevorzugt Glockenböden.

Die Destillationskolonne weist bevorzugt bis zu 20 theoretische Trennböden auf.

Es hat sich als vorteilhaft erwiesen, den entstehenden Ammoniak sofort aus der Reaktionsmischung abzutrennen, bevorzugt während der Reaktion in der Stufe a), so daß eine Belegung durch Ammoniumcarbaminat, welches in minimalen Mengen aus Ammoniak und Kohlendioxid durch Zersetzung von Harnstoff gebildet wird, vermieden werden kann.

In einer besonders bevorzugten Ausführungsform wird zur destillativen Abtrennung von Ammoniak ein sogenannter Flash eingesetzt. Dieser Apparat kann ein Behälter oder eine Kombination von Behälter und Kolonne vorzugsweise eine Kolonne sein, wobei im Kopf der Ammoniak und im Sumpf das Di- oder Polyharnstoff abgezogen werden kann. Im Kopf der Kolonne können-neben dem Ammoniak auch weitere leichter als der Di- oder Polyharnstoff siedende Stoffe enthalten sein. Die Trennung erfolgt in einem Druckbereich von 0,001 bis 1 bar, vorzugsweise bei 0,02 - 0,5 bar und einer Temperatur von 50 bis 180 °C, bevorzugt 80 bis 150 °C.

Falls erforderlich kann die Abtrennung zusätzlich noch durch Durchleiten eines unter den Abtrennbedingungen inerten Gases unterstützt werden, bevorzugt durch Stickstoff.

Um mitgerissene flüssige Bestandteile des Stroms zurückzuhalten, können diese durch nachgeschaltete Dephlegmatoren oder Tröpfchenabscheider aus dem Gasstrom entfernt werden. Es ist aber auch möglich, im abgeleiteten Gasstrom einen oder mehrere partielle Kondensatoren vorzuhalten, in denen mitgerissene Flüssigkeit kondensiert wird.

### c) Abtrennung des Harnstoff-Überschusses

Aus der durch kontinuierlichen Betrieb in der ersten Reaktionsstufe erhaltenen Reaktionsmischung (a) und/oder (b) wird in einem geeigneten Verfahren der eingesetzte Harnstoff gelöst und im Anschluss abgetrennt.

Als Lösungsmittel kommen alle Stoffe in Betracht, in denen Harnstoff gut löslich ist, d.h. eine Löslichkeit von mehr als 200 g Harnstoff/l bei der jeweiligen Lösungstemperatur.

Beispiele dafür sind Alkohole und Wasser.

Beispiele für Alkohole sind Methanol, Ethanol, n-Propanol, iso-Propanol, n-Butanol, sek-Butanol und iso-Butanol. Dabei ist darauf zu achten, daß die Temperatur bei der Abtrennung des Harnstoffs von den Di- und Polyharnstoffen so niedrig ist, daß der Alkohol nicht mit den funktionellen Gruppen reagiert.

Erfindungsgemäß bevorzugt erfolgt das Lösen in Wasser.

Erstaunlicherweise lösen sich die in der Reaktionsstufe (a) gebildeten Diharnstoffe bei Temperaturen von 0 - 60 °C, insbesondere bei 10 - 20 °C sehr schlecht in Wasser, d.h. - weniger als 3 g/l, so dass in der sich diesem Verfahrensschritt anschließenden Trennung der Reaktionsprodukte die Abtrennung des Feststoffes (Di- oder Polyharnstoffe) und der bevorzugt wässrigen Harnstofflösung ohne größere Ausbeuteverluste gelingt.

Dies kann durch eine beliebige fest-flüssig-Trennung erfolgen, beispielsweise Filtration oder Zentrifugation. Denkbar wäre auch eine fraktionierte Kristallisation.

Aus der bevorzugt wässrigen Harnstofflösung wird in einem geeigneten Apparat der Harnstoff zurückgewonnen und erneut der Reaktionsstufe zugeführt. Dies kann bevorzugt durch Rektifikation oder besonders bevorzugt durch einstufige Destillation erfolgen. Als Apparate dafür dienen Flash-, Fallfilm-, Dünnschicht und/oder Kurzwegverdampfer, denen gegebenenfalls eine kurze Kolonne aufgesetzt sein kann. Der Harnstoff wird dann bevorzugt als Schmelze wiedergewonnen und rückgeführt.

Wird bereits Wasser als Lösungsmittel in der ersten Stufe verwendet, kann die verbleibende Lösung direkt in die Stufe a) rückgeführt werden, gegebenenfalls nach einer teilweisen Abtrennung des Lösungsmittels.

Es kann auch sinnvoll sein, zur besseren Entmischung des Gemisches aus Harnstoff und Di- oder Polyharnstoff ein organisches Hilfslösungsmittel zuzugeben, das mit der Harnstofflösung nicht mischbar ist und mit dem sich das Gemisch in eine wäßrige und eine organische Phase trennen lassen. Die Di- und Polyharnstoffe sind in gängigen organischen Lösungsmitteln unter den Reaktionsbedingungen in der Regel nicht oder schlecht löslich.

Ist ein weiteres Lösungsmittel vorhanden, so kann bevorzugt eine Phasenseparation durchgeführt werden. Auch das organische Lösungsmittel kann dann von den Di- oder Polyharnstoffen bevorzugt durch Destillation abgetrennt und erneut in die Stufe (c) eingesetzt werden.

In einer bevorzugten Ausführungsform weisen die Di- oder Polyharnstoffe vor dem Einleiten in die Stufe d) noch einen Gehalt an Wasser und/oder Alkohol, insbesondere von Wasser bis zu 10 Gew%, bevorzugt bis zu 7,5 Gew%, besonders bevorzugt bis zu 5 und insbesondere bis zu 3 Gew% auf. Dies ist insbesondere bevorzugt für Diharnstoffe, die sich von 3-Aminomethyl-3,5,5-trimethylcyclohexylamin ableiten. Ein solcher Restgehalt an Lösungsmittel bewirkt, daß das Reaktionsgemisch flüssig bleibt und somit leichter förderbar ist.

### d) Harnstoffspaltung

Der aus der Reaktionsmischung abgetrennte Di- oder Polyharnstoff wird in einem geeigeten Apparat-thermisch gespalten.

Die aus der Reaktionsstufe (c) erhaltene Di- oder Polyharnstoffe enthaltende Reaktionsmischung wird in einer geeigneten Vorrichtung, bevorzugt lösungsmittelfrei oder in Gegenwart von lediglich geringen Mengen an Wasser und/oder Alkohol in flüssiger Phase in Gegenwart oder bevorzugt Abwesenheit von Katalysatoren bei Temperaturen von 150 bis 450 °C, vorzugsweise 200 bis 400 °C und unter vermindertem Druck von 0,01 - 0,6 bar, vorzugsweise im Bereich von 0,02 - 0,1 bar kontinuierlich thermisch gespalten.

Die Verweilzeiten werden vorzugsweise im Bereich von 0,01 - 20 s gewählt.

Der Umsatz von Polyharnstoff zu Polyisocyanat, vorzugsweise von Diharnstoff zu Diisocyanat, in der Vorrichtung zur thermischen Spaltung kann in Abhängigkeit vom verwendeten Polyharnstoff weitgehend frei gewählt werden und liegt zweckmäßigerweise in einem Bereich von 10 bis 99 Gew.%, vorzugsweise 40 bis 98 Gew.% und besonders bevorzugt 70 bis 95 Gew.% der zugeführten Polyharnstoffmenge.

Der ungespaltene Anteil der Reaktionsmischung, der nicht umgesetzte Polyharnstoffe, Oligoharnstoff-polyharnstoffe, hochsiedende Oligomere und andere wiederverwertbare und unverwertbare Nebenprodukte enthält, wird abgetrennt, kontinuierlich aus der Spaltvorrichtung ausgeschleust (Strom d_{H}) und direkt oder gegebenenfalls nach Umsetzung mit Ammoniak oder Wasser in die Reaktionsstufe (a) zurückgeführt.

Als Katalysatoren zur chemischen Spaltung der Polyharnstoffe finden z.B. die vorgenannten, die Harnstoffbildung katalysierende anorganischen und organischen Verbindungen, Verwendung.

Besonders bewährt und daher vorzugsweise verwendet werden Dibutylzinndilaurat, Eisen-(III)-acetylacetonat, Kobalt-(II)-acetylacetonat, Zinkacetylacetonat, Zirkon tetra-n-butanolat und Zinn-(II)-dioctoat.

Als Spaltvorrichtungen eignen sich beispielsweise zylinderförmige Spaltreaktoren, wie z.B. Röhrenöfen oder vorzugsweise Verdampfer, beispielsweise Dünnschicht- oder Bulkverdampfer, wie z.B. Robertverdampfer, Herbertverdampfer, caddle-typ-Verdampfer, Plattenspalter und vorzugsweise Heizkerzenverdampfer.

In einer bevorzugten Ausführungsform der vorliegenden Erfindung wird die Spaltung in einem Wirbelbettreaktor durchgeführt.

Als Wirbelgut werden dabei übliche Materialien in Form von beispielsweise Pulvern oder Preßlingen, z.B. Kugeln, Ringen, Zylindern oder Tabletten, mit einer bevorzugten Korngröße von 0,01 bis 10 mm, besonders bevorzugt 50 bis 500 µm verwendet.

Die Wirbelschichtanlagen beinhalten üblicherweise einen beispielsweise zylinderförmigen Wirbelbehälter, dessen Innendurchmesser zur Länge (Höhe) im allgemeinen 1 : 0,5 bis 1 : 10, vorzugsweise 1 : 1 bis 1 : 7 beträgt, mit einem Wirbelbett, dem Anströmboden für das Wirbelgas, der beispielsweise aus einer Frittenplatte, einem Rundloch- oder Glockenboden oder einem Conidur-Feinlochblech bestehen kann, einer Düse für die Edukt-, d.h. der Di- oder Polyharnstoffzufuhr und der Produkt- und Abgasabfuhr, die gegebenenfalls über einen im Wirbelbehälter befindlichen Filter oder über Zyklone erfolgen kann, gegebenenfalls einer mechanischen Zerkleinerungsvorrichtung für das Wirbelgut und gegebenenfalls Heizflächeneinbauten. Die Di- oder Polyharnstoffe werden bevorzugt dampfförmig, gelöst oder in geschmolzener Form, besonders bevorzugt gasförmig in die Wirbelschicht eingedüst.

Als Wirbelgas wird bevorzugt Stickstoff eingesetzt, der gegebenenfalls in Mischung mit weiteren Verbindungen verwendet werden kann, beispielsweise mit inerten Substanzen, z.B. Helium, (cyclo)aliphatische und/oder aromatische, gegebenenfalls substituierte Kohlenwasserstoffe mit üblicherweise einem Siedepunkt kleiner 250°C °C, beispielsweise Decan, Undecan, Dodecan, Tridecan, Tetradecan, Naphthalin, Toluol, Benzol, 1, 2-, 1,3- und/oder 1,4-Dimethylbenzol, Chlorbenzoyl, aliphatische und/oder aromatische Ketone, beispielsweise Cyclohexanon. Bevorzugt wird das Wirbelgas durch den Anströmboden und der zu spaltende Di- oder Polyharnstoff durch eine seitliche Düse, die sich in der unteren Hälfte der Wirbelschicht befindet, in den Wirbelbehälter eingeführt.

Als Wirbelgut werden bevorzugt Kohlenstoff und/oder gegebenenfalls gesinterte Oxide, enthaltend Bor, Aluminium, Silizium, Zinn, Blei, Antimon, Zink, Yttrium, Lanthan, Titan, Zirkonium, Niob, Wolfram und/oder Eisen sowie gegebenenfalls Magnesium, Calzium und/oder Phösphor verwendet. Besonders bevorzugt wird als Wirbelgut Siliziumoxide, Kohlenstoff und/oder ein Steatit, besonders bevorzugt Siliziumdioxid oder Steatit mit der allgemeinen Formel Mg₃[SiO₁₀(OH)₂] eingesetzt. Die Verwendung des Steatits bietet den Vorteil, daß während des Prozesses gebildete polymere Belegungen in der Wirbelschicht oder an dem Wirbelbehälter durch Ausbrennen entfernt werden können.

Die Spaltung der Di- oder Polyharnstoffe kann somit bevorzugt derart erfolgen, daß man kontinuierlich getrennt oder gemeinsam Di- oder Polyharnstoff und Wirbelgas in eine Wirbelschicht, bevorzugt mit Siliziumdioxid oder Steatit als Wirbelgut, einführt und Isocyanat und Ammoniak kontinuierlich entfernt und in die Abkühlung (siehe unten) führt.

Die entstehenden Spaltprodukte werden schnell abgekühlt. Als Apparate kommen Wärmetauscher oder Kolonnen in Frage. Die Abkühlung muss dabei schnell erfolgen, da es sonst zu Rekombinationen und damit Verstopfungen kommen kann.

Zur Reduzierung der Konzentration der Spaltprodukte kann ein Lösungsmittel eingesetzt werden oder, falls-die Spaltung in einem Wirbelbett durchgeführt wird, kann für diesen Zweck das Wirbelgas verwendet werden. Der in den Spaltgasen enthaltene Ammoniak wird aus dem Reaktionsgemisch, in dem die entsprechenden Isocyanate bereits in hohen Konzentrationen vorliegen, durch Flashen oder Destillation entfernt.

Die Abkühlung der Spaltprodukte kann indirekt, beispielsweise in Kondensatoren, wie Rohrbündel- oder Plattenkondensatoren, oder bevorzugt direkt erfolgen. Eine bevorzugte Möglichkeit zur direkten Kondensation ist erfindungsgemäß ein sogenannter Quench, also die Abkühlung der Spaltprodukte durch direkten Kontakt mit einem Kühlmedium.

Als Quench-/Vorquenchvorrichtung können alle im Stand der Technik für diesen Zweck bekannten Vorrichtungen, z.B. Sprühkühler, Venturiwäscher, Blasensäulen oder sonstige Apparate mit berieselten Oberflächen, eingesetzt werden, wobei vorzugsweise Venturi-Wäscher oder Sprühkühler verwendet werden.

Erfindungsgemäß bevorzugt ist eine mittlere Abkühlzeit von weniger als 10 Sekunden, bevorzugt weniger als 5 Sekunden, besonders bevorzugt weniger als 3 Sekunden, ganz besonders bevorzugt weniger als 2 Sekunden, insbesondere weniger als 1 Sekunde und speziell weniger als 0,5 Sekunden. Denkbar sind auch Abkühlzeiten von weniger als 0,3, weniger als 0,2 oder sogar 0,1 Sekunden.

Die Abkühlzeit ist dabei definiert als der Zeitraum zwischen dem Zeitpunkt des Entstehens des Spaltproduktgemisches und dem Zeitpunkt, zu dem das Spaltproduktgemisch 90% der Temperaturänderung zur adiabaten Endtemperatur abgeschlossen hat. Durch die gewählten Zeiträume kann ein Verlust von Isocyanat durch Neben- bzw. Weiterreaktionen praktisch vollständig vermieden werden. Die adiabate Endtemperatur ist die Temperatur, die sich einstellt, wenn das Reaktionsgemisch und die Quenchflüssigkeit in den jeweiligen Mengenströmen und Eingangstemperaturen unter adiabatischen Bedingungen vermischt werden.

Bevorzugt wird mindestens ein Quenchmedium, bevorzugt ein flüssiges Quenchmedium mit einer niedrigeren Temperatur als das Spaltgasgemisch in geeigneter Weise mit dem Spaltgasgemisch in Kontakt gebracht. Bevorzugt wird das Quenchmedium in die Quenchzone eingedüst, so daß sich ein Vorhang aus Quenchmedium bildet, durch den das Spaltgasgemisch geführt wird.

Die Flüssigkeitströpfchen des Quenchmediums werden mittels Ein- oder Zweistoffzerstäuberdüsen, vorzugsweise Einstoffzerstäuberdüsen, erzeugt und besitzen vorzugsweise einen Sauter-Durchmesser d₂₃ von 5 bis 5000 µm, besonders bevorzugt 5 bis 500 µm und insbesondere 5 bis 250 µm. Der Sauterdurchmesser d₂₃ beschreibt bis auf einen konstanten Faktor das Verhältnis von Tropfenvolumen zu Tropfenoberfläche (K. Schwister: Taschenbuch der Verfahrenstechnik, Fachbuchverlag Leipzig, Carl Hanser Verlag 2003) und ist somit die für den Quenchprozess wesentliche Kenngröße der erzeugten Tropfengrößenverteilung.

Die Zerstäuberdüsen erzeugen je nach Ausführungsform einen Sprühkegelwinkel von 10 bis 140°, bevorzugt von 10 bis 120°, besonders bevorzugt von 10° bis 100°.

Die Geschwindigkeit, mit der die Tröpfchen aus der Düse austreten, beträgt in der Regel mindestens 5 m/s, bevorzugt mindestens 10 m/s und besonders bevorzugt mindestens 25 m/s. In der Regel beträgt die Geschwindigkeit nicht mehr als 200 m/s, bevorzugt nicht mehr als 100 m/s und besonders bevorzugt nicht mehr als 75 m/s.

Die Anzahl der Zerstäuberdüsen ist nicht begrenzt und kann beispielsweise je Einlaß für das Spaltgasgemisch 1 bis 10, bevorzugt 1 bis 6, besonders bevorzugt 1 bis 4, ganz besonders bevorzugt 1 bis 3 und insbesondere 1 bis 2 betragen.

Bevorzugt wird in die Quenchzone der Austrag aus einer Spaltzone geleitet, es können aber auch die Austräge aus mehreren Spaltzonen über einen oder mehrere Einlässe in eine Quenchzone geführt werden.

Es ist auch möglich, den Austrag aus einer Spaltzone aufzuteilen und über mehrere Einlässe in eine oder mehrere Quenchzonen zu führen.

Die Flüssigkeit, die über die Zerstäuberdüsen eingedüst wird, sollte bevorzugt eine gute Löslichkeit für die Di- oder Polyharnstoffe sowie eine geringe für Ammoniak aufweisen. Vorzugsweise werden organische Lösungsmittel eingesetzt. Insbesondere eingesetzt werden aliphatische oder aromatische Lösungsmittel, die mit Halogenatomen substituiert sein können. Beispiele für derartige Flüssigkeiten sind Toluol, Benzol, Nitrobenzol, Anisol, Chlorbenzol, Dichlorbenzol (ortho, para), Trichlorbenzol, Xylol, Hexan, Diethylisophthalat (DEIP), aromatische und/oder aliphatische Kohlenwasserstoffe enthaltende-Gemische, die einen Siedebereich von 60 bis 200 °C- umfassen können, aber auch Tetrahydrofuran (THF), Dimethylformamid (DMF) und deren Gemische.

Die Temperatur der eingedüsten Flüssigkeit liegt vorzugsweise bei 0 bis 200°C, besonders bevorzugt bei 50 bis 150°C und insbesondere bei 70 bis 120°C, so dass bei der eingedüsten Flüssigkeitsmenge die gewünschte Abkühlung und Kondensation des Di- oder Polyharnstoffes erreicht wird. Dies bedingt das weitgehende Stoppen der Reaktion.

Die Geschwindigkeit des Spaltgases in der Quenchzone ist vorzugsweise größer als 1 m/s, besonders bevorzugt größer als 10 m/s und insbesondere größer als 20 m/s.

Das Masseverhältnis von eingedüster Flüssigkeitsmenge zur Menge der gasförmigen Reaktionsmischung beträgt vorzugsweise 100:1 bis 1:10, besonders bevorzugt 50:1. bis 1:5 und insbesondere 10:1 bis 1:2.

Die Trennung der Spaltprodukte und des Quenchmediums kann in einer Kolonne erfolgen, bei der üblicherweise das Isocyanat in der Seite (d_{M}) und der Ammoniak (d_{L}) am Kopf abgezogen werden. Bevorzugt ist jedoch eine zweimalige partielle Kondensation in Wärmetauschern, wobei je nach abgeführter Wärmeleistung und Siedetemperatur des Lösungsmittels im ersten Wärmetauscher überwiegend Isocyanat und im zweiten überwiegend Quenchmedium partiell kondensiert wird. Ammoniak wird dann als Gasstrom gemeinsam mit Lösungsmittelanteilen abgeführt.

### e) Isocyanatreinigung

Das Rohisocyanatgemisch wird in einer sich anschließenden Destillation von Rekombinationsprodukten, Nebenprodukten und sofern vorhanden dem Lösungsmittel befreit. Die Nebenprodukte werden vorzugsweise in die thermische Spaltung zurückgeführt. Ein Teil kann auch ausgeschleust werden

Die bei der thermischen Spaltung gebildeten Spaltprodukte, die sich vor allem aus Ammoniak, Polyisocyanat, vorzugsweise Diisocyanat, und partiell gespaltenen Polyharnstoffen zusammensetzen, werden in Stufe (e) vorteilhafterweise mit Hilfe einer oder mehrerer Destillationskolonnen, vorzugsweise durch Rektifikation bei Temperaturen von 100 bis 220°C, vorzugsweise 120 bis 170°C und einem Druck von 1 bis 200 mbar, vorzugsweise 5 bis 50 mbar, in Leichtsieder und besonders Ammoniak (e_{L}) und eine rohe Polyisocyanatmischung (e_{M}) mit einem Polyisocyanatgehalt von 85 bis 99 Gew.%, vorzugsweise von 95 bis 99 Gew.% getrennt. Die bei der destillativen Trennung anfallenden höhersiedenden Nebenprodukte (e_{H}) und insbesondere die ungespaltenen und partiell gespaltenen Polyharnstoffe werden vorzugsweise in die Spaltvorrichtung (d) und/oder Rückgewinnung (f) geführt.

Mit dem Index "L" werden hier leichtsiedende Ströme der einzelnen Stufen gekennzeichnet, mit dem Index "H" hochsiedende und mit "M" mittelsiedende.

Die vorzugsweise durch Rektifikation erhaltene rohe Polyisocyanatmischung (e_{M}) kann durch Destillation bei einer Temperatur von 100 bis 180°C und unter einem Druck von 1 bis 50 mbar destillativ gereinigt werden, wobei die einzelnen Fraktionen zurückgeführt oder als Reinprodukt isoliert werden. Wie bereits ausgeführt wurde, wird bei der bevorzugt angewandten Reindestillation die Kopffraktion, die vorzugsweise aus Polyisocyanat, insbesondere Diisocyanat besteht, gegebenenfalls nach Umsetzung der freien Isocyanatgruppen mit Ammoniak in die Reaktionsstufe (a) oder die Ammoniakabtrennung (b) zurückgeführt, die Seitenfraktion, die aus reinem Polyisocyanat, insbesondere Diisocyanat, vorzugsweise mit einer Reinheit von mindestens 98 Gew.%, insbesondere über 99 Gew.% besteht, wird abgeleitet und der Lagerung zugeführt und die Sumpffraktion, die als wesentliche Komponenten die partiell gespaltenen Polyharnstoffe und Polyisocyanate enthält, wird vorzugsweise in die Spaltvorrichtung zur thermischen Spaltung zurückgeführt.

Nach anderen Verfahrensvarianten kann die Sumpffraktion (e_{H}) jedoch auch in die Destillationskolonne (c) zur Trennung von rohem Polyisocyanat und Ammoniak oder in die Reaktionsstufe (a), die Polyharnstoffbildung, zurückgeführt werden. Möglich ist auch eine Teilung der Sumpffraktion in 2 oder 3 Produktströme, wobei diese vorzugsweise in der Polyharnstoffbildung (a) und die Spaltvorrichtung (d) sowie gegebenenfalls in die Destillationskolonne (e) oder in die Rückgewinnung (f) zurückgeführt werden.

### f) Rückgewinnung

Die Umsetzung des Reaktionsaustrages (d_{H}) aus d) und/oder Destillationsrückstände (e_{H}) aus (e) können optional erneut dem Prozess zugeführt werden. Dabei wird mit Ammoniak die in diesem Gemisch enthaltenen Isocyanatgruppen und/oder Harnstoffe oder sonstige reaktive Bestandteile zu gegebenenfalls niedermolekulareren Harnstoffen umgewandelt. Es besteht die Möglichkeit, diese Reaktionen in separaten Reaktoren wie z. B. Mischreaktoren oder Strömungsrohren oder auch in (a) durchzuführen. Für die Ammonolyse der Rückstände sind Temperaturen von 20 - 250 °C, vorzugsweise 50 - 220 °C erforderlich. Die mittleren Verweilzeiten liegen dabei im Bereich von wenigen Sekunden bis Stunden.

Dazu können beispielsweise die Ströme (d_{H}) und/oder (e_{H}) sowie gegebenenfalls ein Teil des Stromes (d_{H}) mit Ammoniak zusammengeführt werden, wobei das Molverhältnis von NCO-Gruppen bzw. deren Äquivalenten, also beispielsweise Harnstoffgruppen, zu Ammoniak bis zu 1:100, bevorzugt bis zu 1:20, besonders bevorzugt bis zu 1:10 beträgt.

Bevorzugt kann die Umsetzung mit überkritischem Ammoniak durchgeführt werden.

Der Ammoniak kann dabei beispielsweise der leichtsiedende Strom (c_{L}) aus der Stufe (c) sein und/oder der ammoniakhaltige Strom (d_{L}) aus der Harnstoffspaltung (d) und/oder auch frischer Ammoniak sein.

Die Reaktionsmischung wird in Gegenwart oder Abwesenheit von Katalysatoren innerhalb von 1 bis 150 min, bevorzugt 3 bis 60 min bei Temperatur von 20 bis 200 °C, bevorzugt 50 bis 170°C bei einem Druck von 0,5 bis 20 bar, bevorzugt 1 bis 15 bar umgesetzt.

Die Umsetzung kann in einer kontinuierlichen Kesselkaskade oder in einem Rohrreaktor bevorzugt einphasig durchgeführt werden.

Als Katalysatoren kommen grundsätzlich alle Verbindungen in Frage, die die Reaktion von NCO- mit Ammoniak fördern. Beispielsweise seien genannt Zinnoctoat, Dibutylzinndilaurat, Zinnchlorid, Zinkdichlorid, Zinn-(II)-dioctoat und Triethylamin. Zumeist sind jedoch keine Katalysatoren erforderlich.

Das erfindungsgemäße Verfahren eignet sich insbesondere zur Herstellung von aliphatischen Diisocyanaten, wie 2-Methylpentan-diisocyanat-1,5, isomeren aliphatischen Diisocyanaten mit 6 Kohlenstoffatomen im Alkylenrest und deren Gemische und vorzugsweise Hexamethylendiisocyanat-1,6 und cycloaliphatischen Diisocyanaten, insbesondere 3-Isocyanatomethyl-3,5,5-trimethylcyclohexylisocyanat nach einer wirtschaftlichen Methode.

Die hergestellten Polyisocyanate eignen sich vorzüglich zur Herstellung von Urethan-, Isocyanurat-, Amid- und/oder Harnstoffgruppen enthaltenden Kunststoffen nach dem Polyisocyanat-Polyadditionsverfahren. Sie finden ferner Verwendung zur Herstellung von mit Urethan-, Biuret- und/oder Isocyanuratgruppen modifizierten Polyisocyanatmischungen. Derartige Polyisocyanatmischungen aus aliphatischen oder cycloaliphatischen Diisocyanaten werden insbesondere zur Herstellung von lichtbeständigen Polyurethanlacken und -überzügen verwendet.

Die folgenden Beispiele sollen die Erfindung erläutern, aber nicht auf diese Beispiele einschränken.

### Beispiel 1

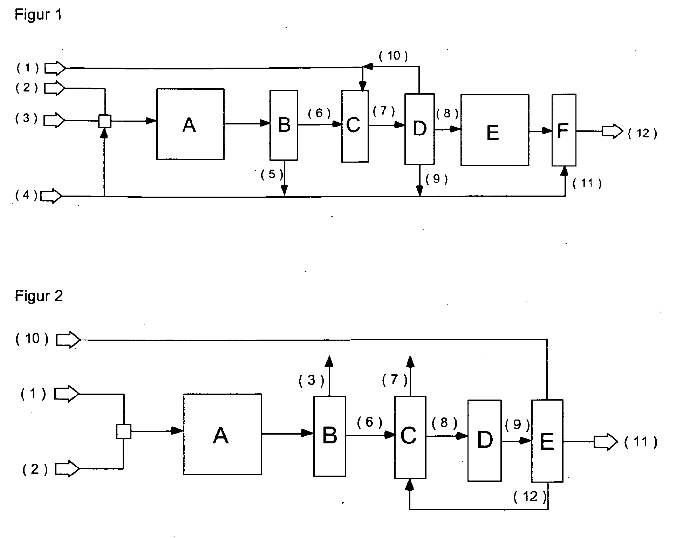

In einem Behälter wird 1,6-Hexamethylendiamin bei einer Temperatur von 90 °C vorgelegt. In einen zweiten Behälter wird-Toluol gefüllt. Einer Mischdüse werden mittels Pumpen ein Strom von 0,9 kg/h 1,6-Hexamethylendiamin ((2) in Figur 1), 8 kg/h Toluol (4) und 6 kg/h flüssiger Harnstoff (3) zugeführt. Die Ströme werden mit Wärmeüberträgern so vorgewärmt, dass in der Düse mindestens eine Temperatur von 130 °C vorliegt.

Das Reaktionsgemisch durchströmt nach Verlassen der Düse dann einen mantelbeheizten Rohrreaktor (A). Die Temperaturen im Reaktor liegen je nach Fahrweise im Bereich von 170 - 220 °C bei einem Druck von 13 bar. Der Reaktor besitzt eine Länge von ca. 8 m. Der Aminumsatz erreicht nahezu 100%. Die Ausbeuten des 1,6-Hexamethylen-di-harnstoffes liegen bei etwa 98%.

Das Reaktionsgemisch aus dem Strömungsrohr wird danach in einem Flash (B) vom größten Teil des Ammoniaks befreit. Die Temperatur im Flash beträgt 110 °C bei einem Druck von ca. 1,1 bar. Etwa die Hälfte des Lösungsmittels wird als Dampf in ein Kondensationssystem mitgerissen. Zwei in Serie geschaltete Kühler sorgen für die Abtrennung des Lösungsmittels vom Ammoniak. Das Toluol wird gesammelt (5) und erneut der Düse zugeführt.

Der flüssige Austrag (6) aus (B) wird einem Mischsystem (C) (Rührreaktor 150 ml) zugeführt. Die Temperatur in diesem Reaktor beträgt 20 °C und der Druck entspricht etwa Atmosphärendruck. In diesen Mischreaktor wird zusätzlich 1 kg/h Wasser (1) dosiert. Der Austrag (7) aus dem Mischer (C) wird einem Phasentrenngefäß (D) zugeführt. Der in der organischen und wässrigen Phase unlösliche 1,6-Hexamethylen-diharnstoff wird als Suspension am Boden mit einer Pumpe kontinuierlich abgezogen (8). Die Oberphase ((9), Toluol) wird ebenfalls mit einer Pumpe kontinuierlich abgezogen, gesammelt und erneut der Reaktionsstufe (A) zugeführt. Das Wasser (10) wird als Mittelphase abgezogen. In diesem Teil ist der überschüssige und in (A) nicht umgesetzte Harnstoff gelöst.

Der wässrige Strom aus dem Phasentrenner (D) wird bei etwa 60 - 80 °C und 300 hPa schonend eingedampft, der Dampf kondensiert und erneut dem Mischer (C) zugeführt. Der als Rückstand verbleibende Harnstoff wird dem Reaktorsystem (A) zugeführt (nicht gezeigt in Figur 1).

Der aus dem Phasentrenngefäß (D) abgezogene 1,6-Hexamethylen-di-harnstoff (8) wird direkt einem Wirbelschichtreaktor (E) zugeführt. Die Temperatur des Reaktors liegt bei 380 °C. Als Wirbelgut wurde Sand (3,5 kg) verwendet. Der Durchmesser des Reaktors beträgt 10 cm. Die den Reaktor verlassenden Brüden wurden in einem Flash (F) mit Toluol (11) schnell abgekühlt, um das entstandene 1, 6-Hexamethylendiisocyanat (HDI, (12)) schnell vom Ammoniak zu trennen. Die Ausbeute an HDI in diesem Verfahrensschritt lag bei 89%. Die sich während der Trennung bildenden Harnstoffe wurden erneut dem Phasentrenngefäß (D) zugeführt (nicht gezeigt in Figur 1).

### Beispiel 2

In einem Behälter wird Isophorondiamin (3-Aminomethyl-3,5,5-trimethylcyclohexylamin) bei einer Temperatur von 90 °C vorgelegt. In einem zweiten Behälter wird Wasser gefüllt und fester Harnstoff in einer Konzentration von 400 g/l gelöst. Einer Mischdüse werden mittels Pumpen ein Strom von 0,9 kg/h Isophorondiamin ((1) in Figur 2), 1,58 I/h Wasser- Harnstoffgemisch (2) zugeführt. Die Ströme werden mit Wärmeüberträgern so vorgewärmt, dass in der Düse mindestens eine Temperatur von 130 °C vorliegt.

Das Reaktionsgemisch durchströmt nach Verlassen der Düse dann einen mantelbeheizten Rohrreaktor (A). Die Temperaturen im Reaktor liegen je nach Fahrweise im Bereich von 170 - 220 °C bei einem Druck von 13 bar. Der Reaktor besitzt eine Länge von ca. 8 m. Der Aminumsatz erreicht nahezu 100%. Die Ausbeuten des Isophoron-di-harnstoffes liegen bei etwa 98%.

Das Reaktionsgemisch aus dem Strömungsrohr wird danach in einem Flash (B) vom größten Teil des Ammoniaks und Wassers befreit. Die Temperatur im Flash beträgt 110 °C bei etwa 1,1 bar. Etwa die Hälfte des Wassers wird als Dampf in ein Kondensationssystem mitgerissen. Zwei in Serie geschaltete Kühler sorgen für die Abtrennung des Lösungsmittels vom Ammoniak. Das Wasser wird gesammelt (3) und erneut dem Vorlagebehälter zugeführt.

Der flüssige Austrag (6) aus (B) wird einem Dünnschichtverdampfer (C) zugeführt. Bei einem Druck von 1 bar wird der größte Teil des restlichen Wassers als Dampfphase abgetrennt (7) und der flüssige Austrag des Di-harnstoffes im Sumpf mittels Pumpe abgezogen (8).

Der aus dem Dünnschichtverdampfer (C) abgezogene flüssige Isophoron-di-harnstoff (8) wird direkt einem Wirbelschichtreaktor (D) zugeführt. Die Temperatur des Reaktors liegt bei 380 °C. Als Wirbelgut wurde Sand (3,5 kg) verwendet. Der Durchmesser des Reaktors beträgt 10 cm. Die den Reaktor verlassenden Brüden (9) wurden in einem Flash (E) mit Toluol (10) schnell abgekühlt, um das entstandene Isophorondiisocyanat (IPDI, (11)) schnell vom Ammoniak zu trennen. Die Ausbeute an IPDI in diesem Verfahrensschritt lag bei 90%. Die sich während der Trennung bildenden Harnstoffe wurden erneut dem Dünnschichtverdampfer (C) zugeführt (12).

## Patentansprüche

1. Verfahren zur Herstellung von Isocyanaten durch Umsetzung mindestens eines Di- oder Polyamins mit Harnstoff zum korrespondierenden Di- oder Polyharnstoff und anschließender thermischer Spaltung der so erhaltenen Di- oder Polyharnstoffe in die korrespondierenden Isocyanate.

2. Mehrstufiges Verfahren zur kontinuierlichen Herstellung von organischen Di- oder Polyisocyanaten gemäß Anspruch 1 durch Umsetzung der entsprechenden primären organischen Di- oder Polyamine mit Harnstoff in die entsprechenden Di- oder Polyharnstoffe und deren thermische Spaltung, wobei man
a) mindestens ein organisches Di- oder Polyamin mit Harnstoff in Gegenwart oder vorzugsweise in Abwesenheit mindestens eines Katalysators und in Abwesenheit eines Alkohols zu den korrespondierenden Di- oder Polyharnstoffen umsetzt,
b) den dabei entstehenden Ammoniak abtrennt,
c) aus dem Austrag aus a) oder b) überschüssigen Harnstoff und gegebenenfalls weitere Nebenkomponenten abtrennt,
d) das vom Harnstoff und gegebenenfalls weiteren Nebenkomponenten befreite Di- oder Polyharnstoffe enthaltende Reaktionsgemisch aus c) zumindest teilweise einer thermischen Spaltung zuführt, in der man das Reaktionsgemisch in einer Spalteinrichtung in das entsprechende Di- oder Polyiisocyanat und Ammoniak spaltet,
e) das aus (d) erhaltene Rohisocyanat in mindestens einer Destillation reinigt und anfallende Destillationsrückstände zumindest teilweise erneut der Spaltung (d) zuführt und/oder mit Ammoniak und/oder Wasser zu Di- oder Polyharnstoff und/oder Amin umwandelt und der Reaktionseinheit (a) und/oder (b) zuführt.

3. Verfahren gemäß einem der vorstehenden Ansprüche, **dadurch gekennzeichnet, daß** das Di- oder Polyamin ausgewählt ist aus der Gruppe bestehende aus 2,4- und 2,6-Toluylen-diamin, 4,4'-, 2,4'- und 2,2'-Diamino-diphenylmethane und die entsprechenden Isomerengemische, Butandiamin-1,4, 2-Ethylbutandiamin-1,4, Octandiamin-1,8, Decandiamin-1,10, Dodecandiamin-1,12, Cyclohexandiamin-1,4, 2-Methyl-, 4-Methyl-cyclohexandiamin-1,3, 1,3- und 1,4-Diaminomethylcyclohexan, 4,4'-Di(aminocyclohexyl)methan, 3 (bzw. 4), 8 (bzw. 9)-Bis(aminomethyl)-tricyclo[5.2.1.0^{2.6}]decan-Isomerengemische, 2-Methylpentandiamin-1,5, 2,2,4- bzw. 2,4,4-Trimethylhexandiamin-1,6, Hexandiamin-1,6 und 3-Aminomethyl-3,5,5-trimethylcyclohexylamin.

4. Verfahren gemäß einem der Ansprüche 2 bis 3, **dadurch gekennzeichnet, daß** der überschüssige Harnstoff in Schritt (c) mit Wasser aus dem Reaktionsgemisch gelöst wird.

5. Verfahren gemäß einem der Ansprüche 2 bis 4, **dadurch gekennzeichnet, daß** die thermische Spaltung der Di- oder Polyharnstoffe in Schritt (d) in einem Spaltreaktor durchgeführt wird, ausgewählt aus der Gruppe bestehend aus Röhrenöfen, Dünnschichtverdampfer, Bulkverdampfer, Robertverdampfer, Herbertverdampfer, caddle-typ-Verdampfer, Plattenspalter, Heizkerzenverdampfer und Wirbelbettreaktor.

6. Verfahren gemäß Anspruch 5, **dadurch gekennzeichnet, daß** ein Wirbelbettreaktor eingesetzt wird mit einem Wirbelgut ausgewählt aus der Gruppe bestehend aus Siliziumoxid, Kohlenstoff und Steatit.

7. Verfahren gemäß einem der Ansprüche 2 bis 6, **dadurch gekennzeichnet, daß** das in den Schritt (d) geleitete Di- oder Polyharnstoff einen Gehalt an Wasser und/oder Alkohol bis zu 10 Gew% aufweist.

8. Verfahren gemäß einem der Ansprüche 2 bis 7, **dadurch gekennzeichnet, daß** das im Schritt (d) gebildete Spaltproduktgemisch eine mittlere Abkühlzeit von weniger als 10 Sekunden aufweist.

9. Verfahren gemäß einem der Ansprüche 2 bis 8, **dadurch gekennzeichnet, daß** das im Schritt (d) gebildete Spaltproduktgemisch mit einem Quenchmedium abgekühlt wird.

10. Verfahren gemäß Anspruch 9, **dadurch gekennzeichnet, daß** das Quenchmedium im Schritt (d) ausgewählt ist aus der Gruppe bestehend aus Toluol, Benzol, Nitrobenzol, Anisol, Chlorbenzol, Dichlorbenzol (ortho, para), Trichlorbenzol, Xylol, Hexan, Diethylisophthalat (DEIP), aromatische und/oder aliphatische Kohlenwasserstoffe enthaltenden Gemischen, die einen Siedebereich von 60 bis 200 °C umfassen können, Tetrahydrofuran (THF), Dimethylformamid (DMF).

11. Verfahren gemäß Anspruch 9 oder 10, **dadurch gekennzeichnet, daß** das Quenchmedium im Schritt (d) eine Temperatur von 0 bis 200 °C aufweist.

## Claims

1. A process for the preparation of isocyanates by reaction of at least one di- or poly-amine with urea to form the corresponding di- or poly-urea followed by thermal decomposition of the resulting di- or poly-ureas to produce the corresponding isocyanates.

2. A multiple-stage process for the continuous preparation of organic di- or poly-isocyanates according to claim 1 by reaction of the corresponding primary organic di- or poly-amines with urea to form the corresponding di- or poly-ureas and the thermal decomposition thereof, wherein
a) at least one organic di- or poly-amine is caused to react with urea in the presence of, or preferably in the absence of, at least one catalyst and in the absence of an alcohol to form the corresponding di- or poly-ureas,
b) the resultant ammonia is removed,
c) excess urea and, any other minor constituents are separated off from the effluent from a) or b),
d) the reaction mixture comprising di- or poly-ureas freed from the urea and any other minor constituents coming from c) is fed, at least in part, to a thermal decomposition stage, in which the reaction mixture is decomposed in a disintegrator to form the corresponding di- or poly-isocyanate and ammonia,
e) the crude isocyanate obtained in (d) is purified in at least one distillation stage and the resulting distillation residues are at least in part returned to the decomposition stage (d) and/or converted to di- or poly-urea and/or amine with ammonia and/or water and recycled to the reaction unit (a) and/or (b).

3. The process according to any one of the previous claims, wherein the di- or poly-amine is selected from the group consisting of 2,4- and 2,6-toluenediamine, 4,4'-, 2,4'- and 2,2'-diaminodiphenylmethane and the corresponding mixtures of isomers, butanediamine-1,4, 2-ethylbutanediamine-1,4, octanediamine-1,8, decanediamine-1,10, dodecanediamine-1,12, cyclohexanediamine-1,4, 2-methyl-, 4-methylcyclohexanediamine-1,3, 1,3- and 1,4-diaminomethylcyclohexane, 4,4'-di(aminocyclohexyl)methane, 3,8- and 4,9-bis(aminomethyl)-tricyclo[5.2.1.0^{2.6}]decane isomer mixtures, 2-methylpentanediamine-1,5, 2,2,4- or 2,4,4-trimethylhexanediamine-1,6, hexanediamine-1,6, and 3-aminomethyl-3,5,5-trimethylcyclohexylamine.

4. The process according to any one Claims 2 to 3, wherein the excess urea in step (c) is dissolved out of the reaction mixture with water.

5. The process according to any one of Claims 2 to 4, wherein the thermal decomposition of di- or poly-ureas in step (d) is carried out in a breakdown reactor selected from the group consisting of tubular ovens, thin-film evaporators, bulk evaporators, Robert evaporators, Herbert evaporators, Caddle-type evaporators, plate reactors, glow-plug evaporators, and fluidized-bed reactors.

6. The process according to Claim 5, wherein a fluidized-bed reactor is used employing a fluidized medium selected from the group consisting of silicon oxide, carbon, and steatite.

7. The process according to any one of Claims 2 to 6, wherein the di- or poly-urea passed to step (d) has a content of water and/or alcohol of not more than 10% by weight.

8. The process according to any one of Claims 2 to 7, wherein the breakdown product mixture formed in step (d) has an average cooling time of less than 10 seconds.

9. The process according to any one Claims 2 to 8, wherein the breakdown product mixture formed in step (d) is cooled with a quenching medium.

10. The process according to claim 9, wherein the quenching medium in step (d) is selected from the group consisting of toluene, benzene, nitrobenzene, anisole, chlorobenzene, dichlorobenzene (ortho, para), trichlorobenzene, xylene, hexane, diethyl isophthalate (DEIP), aromatic and/or aliphatic hydrocarbon-containing mixtures, which may comprise a boiling range of from 60 to 200°C, tetrahydrofuran (THF), and dimethylformamide (DMF).

11. The process according to claim 9 or 10, wherein the quenching medium in step (d) has a temperature of from 0 to 200°C.

## Revendications

1. Procédé pour la préparation d'isocyanates par transformation d'au moins une diamine ou polyamine avec de l'urée en diurée ou polyurée correspondante et dissociation thermique consécutive des diurées ou polyurées ainsi obtenues en isocyanates correspondants.

2. Procédé en plusieurs étapes pour la préparation continue de diisocyanates ou polyisocyanates organiques selon la revendication 1 par transformation des diamines ou polyamines organiques primaires correspondantes avec de l'urée en diurées ou polyurées correspondantes et leur dissociation thermique, où
a) on transforme au moins une diamine ou polyamine organique avec de l'urée en présence ou de préférence en l'absence d'au moins un catalyseur et en l'absence d'un alcool en diurées ou polyurées correspondantes,
b) on sépare l'ammoniaque qui se forme,
c) on sépare l'urée en excès et le cas échéant d'autres composants secondaires du produit évacué de a) ou de b),
d) on alimente le mélange réactionnel de c) contenant des diurées ou des polyurées, libéré de l'urée et le cas échéant des autres composants secondaires, au moins partiellement dans une dissociation thermique dans laquelle on dissocie le mélange réactionnel dans un dispositif de dissociation en diisocyanate ou polyisocyanate correspondant et en ammoniaque,
e) on purifie l'isocyanate brut obtenu de (d) dans au moins une distillation et on alimente à nouveau les résidus de distillation produits au moins partiellement dans la dissociation (d) et/ou on les transforme avec de l'ammoniaque et/ou de l'eau en diurée ou polyurée et/ou amine et on les alimente dans l'unité de réaction (a) et/ou (b).

3. Procédé selon l'une quelconque des revendications précédentes, **caractérisé en ce que** la diamine ou la polyamine est choisie dans le groupe constitué par la 2,4-toluylènediamine et la 2,6-toluylènediamine, le 4,4'-diaminodiphénylméthane, le 2,4'-diaminodiphénylméthane et le 2,2'-diaminodiphénylméthane et les mélanges d'isomères correspondants, la butanediamine-1,4, la 2-éthylbutanediamine-1,4, l'octanediamine-1,8, la décanediamine-1,10, la dodécanediamine-1,12, la cyclohexanediamine-1,4, la 2-méthylcyclohexanediamine-1,3, la 4-méthylcyclohexanediamine-1,3, le 1,3-diaminométhylcyclohexane et le 1,4-diaminométhylcyclohexane, le 4,4'-di(aminocyclohexyl)méthane, les mélanges de 3(ou, selon le cas, 4),8(ou, selon le cas, 9)-bis(aminométhyl)-tricyclo[5,2,1,0^{2,6}]décanes isomères, la 2-méthylpentanediamine-1,5, la 2,2,4-triméthylhexanediamine-1,6 ou la 2,4,4-triméthylhexanediamine-1,6, l'hexanediamine-1,6 et la 3-aminométhyl-3,5,5-triméthylcyclohexylamine.

4. Procédé selon l'une quelconque des revendications 2 à 3, **caractérisé en ce que** l'urée en excès dans l'étape (c) est éliminée par dissolution avec de l'eau du mélange réactionnel.

5. Procédé selon l'une quelconque des revendications 2 à 4, **caractérisé en ce que** la dissociation thermique des diurées ou polyurées dans l'étape (d) est réalisée dans un réacteur de dissociation choisi dans le groupe constitué par les fours tubulaires, les évaporateurs en couche mince, les évaporateurs pour gros volumes, les évaporateurs de Robert, les évaporateurs d'Herbert, les évaporateurs de type caddle, les dispositifs de dissociation à plaques, les évaporateurs à bougies chauffantes et les réacteurs à lit fluidisé.

6. Procédé selon la revendication 5, **caractérisé en ce qu'**on utilise un réacteur à lit fluidisé, présentant un produit fluidisé choisi dans le groupe constitué par l'oxyde de silicium, le carbone et la stéatite.

7. Procédé selon l'une quelconque des revendications 2 à 6, **caractérisé en ce que** la diurée ou la polyurée guidée dans l'étape (d) présente une teneur en eau et/ou en alcool jusqu'à 10% en poids.

8. Procédé selon l'une quelconque des revendications 2 à 7, **caractérisé en ce que** le mélange de produits dissociés formé dans l'étape (d) présente un temps de refroidissement moyen de moins de 10 secondes.

9. Procédé selon l'une quelconque des revendications 2 à 8, **caractérisé en ce que** le mélange de produits dissociés formé dans l'étape (d) est refroidi avec un agent de désactivation.

10. Procédé selon la revendication 9, **caractérisé en ce que** l'agent de désactivation dans l'étape (d) est choisi dans le groupe constitué par le toluène, le benzène, le nitrobenzène, l'anisol, le chlorobenzène, le dichlorobenzène (ortho, para), le trichlorobenzène, le xylène, l'hexane, l'isophtalate de diéthyle (DEIP), les mélanges contenant des hydrocarbures aromatiques et/ou aliphatiques, qui peuvent présenter une plage d'ébullition de 60 à 200°C, le tétrahydrofuranne (THF), le diméthylformamide (DMF).

11. Procédé selon la revendication 9 ou 10, **caractérisé en ce que** l'agent de désactivation dans l'étape (d) présente une température de 0 à 200°C.
